Europäisches Patentamt

European Patent Office

Office européen des brevets

(11) Veröffentlichungsnummer: **0 596 421 A1**

## (12) EUROPÄISCHE PATENTANMELDUNG

(21) Anmeldenummer: 93117547.5

(22) Anmeldetag: 29.10.93

(51) Int. Cl.5: **G01N 21/55**, G01N 33/551, G01N 33/547

(30) Priorität: 06.11.92 CH 3444/92

(43) Veröffentlichungstag der Anmeldung:
**11.05.94 Patentblatt 94/19**

(84) Benannte Vertragsstaaten:
**BE CH DE DK ES FR GB GR IE IT LI LU NL PT**

(71) Anmelder: **F. HOFFMANN-LA ROCHE AG**
**Postfach 3255**
**CH-4002 Basel(CH)**

(72) Erfinder: **Barner, Richard**
**16 Traubenweg**
**CH-4108 Witterswil(CH)**
Erfinder: **Fattinger, Christof**
**7 Emmengasse**
**CH-4249 Blauen(CH)**
Erfinder: **Huber, Walter**
**62 Ziegelhofweg**
**CH-4303 Kaiseraugst(CH)**
Erfinder: **Hübscher, Josef**
**1 Säspelstrasse**
**CH-4208 Nunningen(CH)**
Erfinder: **Schlatter, Daniel**
**67 Bruderholzstrasse**
**CH-4104 Oberwil(CH)**

(74) Vertreter: **Buntz, Gerhard et al**
**Grenzacherstrasse 124**
**Postfach 3255**
**CH-4002 Basel (CH)**

(54) **Biologisch erkennende Elemente auf neuen TiO2-Wellenleitern für die Anwendung in der Biosensorik.**

(57) Die vorliegende Erfindung betrifft die Beschichtung dielektrischer $TiO_2$-Wellenleiter mit biologisch erkennenden Elementen, wodurch Biosensoren hoher Empfindlichkeit und Spezifität für ein Analytmolekül entstehen.

Die Beschichtung besteht aus einer organischen Trägerschicht, an die die Rezeptormoleküle gebunden sind, wobei die Trägerschicht eine geordnete monomolekulare Schicht beinhaltet, welche aus Molekülen der allgemeinen Formel I

$$\geqslant Si - Y - Z \qquad I$$

besteht.

Diese Schicht ist über das Si-Atom direkt an einen $TiO_2$-Wellenleiter oder gewünschtenfalls über eine Zwischenschicht an einen $TiO_2$-Wellenleiter gebunden.

Bei den Rezeptormolekülen handelt es sich um biologische Moleküle mit Erkennungseigenschaften wie Antigene, Antikörper, Rezeptoren, dsDNA, ssDNA.

Die Anordnung der Rezeptormoleküle auf der Sensoroberfläche kann sowohl zweidimensional als auch dreidimensional sein, und die Rezeptormoleküle können ungerichtet oder gerichtet an der organischen Trägerschicht immobilisiert sein.

EP 0 596 421 A1

Die vorliegende Erfindung betrifft optische Biosensoren sowie Verfahren zu deren Herstellung; im Speziellen betrifft sie Methoden und Verbindungen zum Aufbringen von biologisch erkennenden Elementen auf die für diese neuen optischen Sensoren verwendeten $TiO_2$-Wellenleiter.

Gemäss Definition ist ein Biosensor ein Gerät bestehend aus einem Transducer und einem biologisch erkennenden Element (Trends in Biotechnol. 2 (1984), 59). Derartige Biosensoren können eingesetzt werden für die Bestimmung von Analytkonzentrationen z.B. in der Human- und Veterinärdiagnostik, in der Umweltanalytik und in der Lebensmittelanalytik oder innerhalb der biochemischen Forschung für die Quantifizierung von intermolekularen Wechselwirkungen biologisch aktiver Substanzen (z.B. Antikörper-Antigen-Wechselwirkung, Rezeptor-Ligand-Wechselwirkung, DNA-Protein-Wechselwirkung etc.).

Dem biologisch erkennenden Element eines Biosensors kommt die Aufgabe zu, ein Analytmolekül in Lösung zu erkennen und zu binden (sog. Affinitätssensor) oder katalytisch umzuwandeln (sog. erzymatischer, metabolischer Sensor). Die damit einhergehende Veränderung des biologisch erkennenden Elementes wird durch den mit dem Element in engem Kontakt stehenden Transducer (Signalwandler) erkannt und in ein prozessierbares Signal umgewandelt.

Eine Kasse derartiger Transducer detektiert Aenderungen in den optischen Eigenschaften des biologisch erkennenden Elementes (z.B. Absorption, Brechzahl) mittels einer optischen Oberflächenwelle, die in einer wellenleitenden Schicht entlang der Grenzfläche Transducer/erkennendes Element geführt wird. Diese Klasse von Transducern lässt sich aufgrund von unterschiedlichen wellenleitenden Schichtstrukturen in zwei Gruppen einteilen. Eine erste Gruppe umfasst Transducer, bei denen diese wellenleitende Struktur die Grenzfläche zwischen einem Metall und einem Dielektrikum darstellt. Die an dieser Grenzfläche geführte Welle ist das Oberflächenplasmon (Sensors and Actuators 4 (1983) 299). Die zweite Gruppe umfasst Transducer mit dielektrischen wellenleitenden Schichten. Die geführten optischen Wellen sind Wellenleitermoden (Opt. Lett. 9 (1984) 137; Sensors and Actuators A, 25 (1990) 185; Sensors and Actuators B, 6 (1992) 122; Proc. Biosensors 92, extended abstracts, pp 339 & pp 347).

Die vorliegende Erfindung betrifft Biosensoren, denen dielektrische Wellenleiter zugrunde liegen. Das Funktionsprinzip derartiger Transducer kann auf der Basis der Feldverteilung der in derartigen Wellenleitern geführten Moden erklärt werden. Das elektrische Feld der geführten Moden ist nicht allein auf die geometrischen Abmessungen des Wellenleiters beschränkt, sondern besitzt sogenannte evanescente Anteile, d.h. die Feldverteilung der geführten Moden klingt in den zum Wellenleiter benachbarten Medien (z.B. in dem die wellenleitende Schicht tragenden Substrat oder im Superstrat) exponentiell ab. Veränderungen in den optischen Eigenschaften des dem Wellenleiter benachbarten Substrates oder Superstrates innerhalb der Reichweite des evanescenten Feldes beeinflussen die Propagation dieser Moden und können durch geeignete Messeinrichtungen detektiert werden. Enthält das Superstrat innerhalb der Reichweite des evanescenten Feldes das biologisch erkennende Element, so können die mit dem Binden oder Umwandeln einhergehenden Veränderungen in den optischen Eigenschaften dieses Elementes durch diese optische, oberflächensensitive Methode detektiert und in Bezug auf eine Analytkonzentration kalibriert werden.

Es ist aus der Literatur bekannt, dass die Oberflächenspezifizität und die Oberflächensensitivität des Verfahrens umso höher sind, je kleiner die effektive Dicke der wellenleitenden Schicht ist (Monomode-Wellenleiter) und je höher der Brechzahlsprung an der Grenzfläche Wellenleiter/Substrat und Wellenleiter/Superstrat ist. $TiO_2$ ist aufgrund seiner hohen Brechzahl deshalb als Material für derartige Wellenleiter besonders geeignet und es konnte kürzlich gezeigt werden (Proc. Materials res.Soc. Spring Meeting, San Francisco, 1992), dass mittels der PICVD Technik wellenleitende Filme aus diesem Material mit einer Brechzahl von 2.45 für die Sensorik hergestellt werden können.

Die vorliegende Erfindung betrifft die Beschichtung derartiger $TiO_2$-Wellenleiter mit biologisch erkennenden Elementen, wodurch Biosensoren hoher Empfindlichkeit und Spezifizität für ein Analytmolekül entstehen. Die Grundlage für diese erkennenden Elemente liegt darin, dass für ein selektives Erkennen und Binden (und/oder Umwandeln) eines Analytmoleküls (z.B. Antigen, Ligand, Wirkstoff, ssDNA, etc) ein sogenanntes Erkennungsmolekül (z.B. Antikörper, Membranrezeptor, ssDNA-Probe etc) herangezogen wird. Diese Erkennungsmoleküle können dabei sowohl in ihrer natürlich vorkommenden und isolierbaren Form als auch in einer chemisch oder biotechnologisch herstellbaren Form verwendet werden.

Die Aufgabe der vorliegenden Erfindung liegt darin, einen optischen Biosensor bereitzustellen sowie ein Verfahren zu seiner Herstellung, wobei der Biosensor auf einem $TiO_2$-Wellenleiter und einer organischen Trägerschicht mit den daran gebundenen Rezeptormolekülen besteht und diese Trägerschicht den Anforderungen der optischen Biosensorik genügt, d.h.

- deren Schichtdicke nicht grösser ist als die Reichweite des evanescenten Feldes des im Wellenleiter geführten Modes

- deren Aufbau sich in Bezug auf das im Wellenleiter geführte Licht als optisch homogen erweist
- welche sich als chemisch resistent erweist gegen Medien mit denen sie in Kontakt kommt (Seren, Fermentationslösungen, etc.)
- an welcher die Erkennungsmoleküle so verankert sind, dass sie ihre natürliche Aktivität bewahren
- an welcher die Erkennungsmoleküle so verankert sind, dass sie im Kontakt mit der Probe nicht wegdissozieren.

Entsprechend den verschiedenen Analytmolekülen bzw. erkennenden Rezeptormolekülen sind als Aufgabe der Erfindung entsprechende erkennende Elemente auf den neuen $TiO_2$-Wellenleitern bereitzustellen.

Erfindungsgemäss wird die Aufgabe gelöst durch Bereitstellen, eines optischen Biosensors bestehend aus einem dielektrischen Wellenleiter und einer organischen Trägerschicht an welche Rezeptormoleküle gebunden sind, wobei die organische Trägerschicht und die daran gebundenen Rezeptormoleküle eine geordnete monomolekulare Schicht bildet und wobei die Trägerschicht aus Molekülen der allgemeinen Formel I

$$\succ Si - Y - Z \qquad I$$

besteht und wobei die Moleküle der Trägerschicht über das Si-Atom direkt an einen $TiO_2$-Wellenleiter gebunden sind oder gewünschtenfalls über eine Zwischenschicht an einen $TiO_2$-Wellenleiter gebunden sind.

Im folgenden werden Beispiele von optischen Biosensoren sowie ein Verfahren zur Darstellung der erfindungsgemässen Biosensoren beschrieben.

Für den Aufbau der organischen Trägerschichten mit den hier geforderten Eigenschaften wird die $TiO_2$-Wellenleiteroberfläche zuerst mit einer homogenen organischen Zusatzschicht versehen. Die für die Derivatisierung der $TiO_2$-Oberfläche verwendeten Verbindungen sind Silane der allgemeinen Formel II

$$(R^1R^2R^3)Si\text{-}Y\text{-}X \qquad II$$

worin

$-Si(R^1R^2R^3)$ eine Kopplungsgruppe zur $TiO_2$-Schicht darstellt und $R^1R^2R^3$ Alkyl, Alkoxy oder Halogen sein kann, jedoch mindestens einer dieser Reste entweder Alkoxy oder Halogen ist,

-Y eine Spacergruppe ist und als solche entweder eine Alkylenkette $-CH_2-(CH_2)_n-CH_2-$, eine Fluoralkylenkette $-CH_2-(CF_2)_n-CH_2-$ oder $-CH_2-(CF_2)_n-CF_2-$ mit n = 1-30, eine Oligoethylenkette $-[(CH_2)_{n'}-O-(CH_2)_{n''}]m-$ mit n',n'' = 2-6 und m = 2-6 oder eine Kombination von Alkylen-, Fluoralkylen oder Oligoalkylenglykol darstellt und

-X entweder Wasserstoff oder Fluor oder eine chemisch reaktive Gruppe ist, welche mit $-Si(R^1R^2R^3)$ kompatibel ist wie z.B Karbonsäurehalogenid (-COHal), Olefin ($-CH = CH_2$), Nitril (-CN), Thiocyanat (-SCN) und Thioacetat ($-SCOCH_3$) oder wenn $R^1, R^2, R^3$ = Alkoxy auch Amin ($-NH_2$).

Diese Gruppen X können nach der Addition der Verbindungen an die $TiO_2$-Oberfläche durch geeignete chemische Nachbehandlungen auch überführt werden in nicht $-Si(R^1R^2R^3)$-kompatible Gruppen wie z.B. in Azid und weiter in Amin, oder Nitril in Amin, oder Halogen in Thiocyanat und weiter in Thiol, oder Thioacetat in Thiol oder Olefin in Epoxid, Diol, Halogenid, Dihalogenid oder Karbonsäure etc.

An die ursprüngliche Gruppe X oder die wie eben beschrieben nachbehandelte Gruppe X können weitere Moleküle angekoppelt werden, sodass eine organische Trägerschicht entsteht an die die Rezeptormoleküle gebunden werden.

So entsteht ein optischer Biosensor, wobei die organische Trägerschicht geordnete monomolekulare Schichten bildet, wobei die Trägerschicht aus Molekülen der allgemeinen Formel I besteht.

$$\succ Si - Y - Z \qquad I$$

Dabei bedeuten die Gruppen Z:
- Hydroxyl-, Karboxyl-, Amin, Methyl-, Alkyl-, Fluoralkyl-Gruppen
- Derivate von hydrophilen kurzkettigen Molekülen wie Oligovinylalkoholen, Oligoacrylsäuren, Oligoethylenglycolen
- Derivate von mono- oder oligo-Sacchariden mit 1-7 Zuckereinheiten
- Derivate von Karboxyglycosiden
- Derivate von Aminoglycosiden wie Fradiomycin, Kanamycin, Streptomycin, Xylostasin, Butirosin
- Derivate von Hydrogel bildenden Gruppen natürlichen oder synthetischen Ursprungs wie Dextran, Agarose, Algininsäure, Stärke, Zellulose und Derivate derartiger Polysaccharide oder hydrophile Polymere wie Polyvinylalkohole, Polyacrylsäuren, Polyethylenglykole und Derivate derartiger Polymere

Ueberraschenderweise wurde gefunden, dass sich die Verbindungen der Formel II in ausgezeichneter Art und Weise dazu eignen, um monomolekulare, dicht gepackte, geordnete organische Filme mit der für die optische Sensorik geforderten Qualität auf $TiO_2$ aufzubringen.

Für niedermolekulare Vertreter der Verbindungen mit Formel II erfolgt diese Beschichtung bevorzugt aus der Gasphase (Chemical Vapor Deposition (CVD)- Verfahren.) Für hochmolekulare Verbindungen kann diese Beschichtung aus der flüssigen Phase erfolgen. Um jedoch bei der Beschichtung des $TiO_2$-Wellenleiters eine für die Anwendung in der Optik ausreichende Homogenität zu erreichen, müssen die verwendeten Lösungsmittel auf die Verbindungen der Formel II abgestimmt werden.

Die biologisch erkennenden Elemente in der Biosensorik sind im allgemeinen aufgebaut aus einer mit dem Substrat (Transduceroberfläche) kovalent verknüpften, organischen Trägerschicht, an welche biologische Erkennungsmoleküle adsorptiv oder bevorzugterweise kovalent gebunden sind. Wie nachfolgend gezeigt wird, ist innerhalb der Biosensorik die spezifische Auslegung eines biologisch erkennenden Elementes einerseits eng verknüpft mit der Art des nachzuweisenden Analytmoleküls und dadurch mit der Art des für den Nachweis einzusetzenden Rezeptormoleküls und andererseits aber auch abhängig von der mit einem bestimmten Biosensor für ein Rezeptor-/Analytmolekül Paar zu lösenden Problemstellung.

Die biologisch erkennenden Elemente, wie sie hier für die Verwendung in der optischen Biosensorik in Kombination mit $TiO_2$-Wellenleitern beansprucht werden, gliedern sich in die zwei Hauptklassen A und B, wobei jede dieser Hauptklassen in zwei Unterklassen A1,A2 und B1, B2 unterteilt werden kann. Das für die Zuteilung eines Erkennungselementes zu einer der Hauptklassen relevante Kriterium betrifft die Anordnung der Rezeptormoleküle auf der Sensoroberfläche. Innerhalb der ersten Klasse (A) sind die Rezeptormoleküle annähernd in einer Ebene (zweidimensionale Anordnung) auf der Oberfläche des optischen Transducers angeordnet. Eine derartige zweidimensionale Anordnung der Rezeptormoleküle ergibt sich nur dann, wenn die zur Transduceroberfläche senkrechte Dimension der organischen Trägerschicht nicht wesentlich grösser ist als die molekularen Abmessungen des an dieser Trägerschicht gebundenen Rezeptormoleküls. Innerhalb der Klasse B erkennender Elemente weisen die immobilisierten Rezeptoren eine dreidimensionale Anordnung auf. Diese dreidimensionale Anordnung kann nur mit einer Trägerschicht realisiert werden, welche eine Dicke aufweist, die wesentlich grösser ist als die molekularen Dimensionen des Rezeptormoleküls und welche sich für das Rezeptormolekül als durchlässig erweist. Diese Art Trägerschicht kann als poröse, dreidimensionale Matrix bezeichnet werden.

Das Kriterium für die Zuteilung zu einer der Subklassen (A1,B1 bzw. A2,B2) betrifft die Art und Weise wie die Rezeptormoleküle an der Trägerschicht immobilisiert sind; wir unterscheiden eine ungerichtete (A1,B1) und eine gerichtete Art (A2,B2) des Immobilisierens, wobei der Begriff Immobilisieren sowohl für ein adsorptives als auch für ein kovalentes Binden an die organische Trägerschicht verwendet wird. Ungerichtetes Immobilisieren eines Rezeptormoleküls an der organischen Trägerschicht bedeutet, dass beim Verbinden des Rezeptormoleküls mit der organischen Trägerschicht keine Rücksicht auf bestimmte Strukturmerkmale des Rezeptormoleküls genommen wird, d.h. die Immobilisierung erfolgt über beliebige Stellen auf der Oberfläche des Rezeptormoleküls. Gerichtetes Immobilisieren bedeutet, dass beim Immobilisieren des Rezeptormoleküls auf die Analyterkennenden Domänen Rücksicht genommen wird und für das Immobilisieren derartige Strukturelemente ausgewählt werden, welche räumlich von den Analyt-erkennenden Domänen gut separiert sind.

Wie einleitend angesprochen, hat jede dieser unterschiedlichen Arten biologisch, erkennender Elemente ihre spezifische Eignung für die Anwendung in unterschiedlichen Gebieten oder Fragestellungen der Bioanalytik. Dies soll mit einigen Beispielen untermauert werden:

Eine dreidimensionale Matrix erlaubt die Immobilisierung einer grösseren Zahl von Rezeptormolekülen pro Flächeneinheit. Da die Gesamtzahl von Rezeptormolekülen pro Flächeneinheit bei direkten Biosensoren die Steilheit der Sensorkennlinie und somit das Auflösungsvermögen in dem für den Sensor relevanten Konzentrationsbereich bestimmt, wird man für eine exakte Bestimmung einer Analytkonzentration (z.B. in der diagnostischen Anwendung) deshalb einen Sensor bevorzugt mit einem dreidimensionalen erkennenden Element ausstatten. Eine dreidimensionale Matrix kann aber von Nachteil sein, wenn das nachzuweisende Analytmolekül über mehrere repetitive Epitope verfügt. Dann führt das Binden dieses Analytmoleküls an mehrere Rezeptormoleküle in den äusseren Regionen des Elementes zu einem Quervernetzen der Trägerschicht, wodurch der Zugang zu freien Bindungsstellen im Innern des erkennenden Elementes für nachfolgende Analytmoleküle erschwert wird. Der Nachteil einer dreidimensionalen Anordnung von Rezeptormolekülen wird auch offensichtlich bei einer quantitativen Beschreibung der Kinetik des Bindungsvorganges zwischen einem Rezeptormolekül und einem Analytmolekül. Die bei einer derartigen Untersuchung beobachtete zeitabhängige Sensorantwort kann beim Verwenden einer dreidimensionalen Matrix auch durch die in dieser Matrix behinderte Diffusion des Analytmoleküls geprägt sein.

In analoger Weise lassen sich auch Vor- und Nachteile eines gerichteten oder ungerichteten Immobilisierens innerhalb unterschiedlicher bioanalytischer Fragestellungen aufzeigen. Für den Nachweis eines Antigens in der Immundiagnostik ist es ohne Zweifel wichtig, den für den Nachweis verwendeten Antikörper so auf der Oberfläche zu immobilisieren, dass die Antigen-erkennenden Domänen nicht durch die Immobilisierung beeinflusst werden, z.B. über den von den Antigen-erkennenden Domänen gut separierten Fc-Teil. Wird jedoch mit einem Biosensor auf die Anwesenheit eines Ensembles von polyklonalen Antikörpern gegen ein und dasselbe Antigen getestet, so ist es zweckmässig das Antigen in ungerichteter Weise zu immobilisieren, damit alle Epitope des Antigens für ein Erkennen durch die Antikörper in Lösung gleichberechtigt sind.

Als eine weitere Ausführungsform der Erfindung ergibt sich demnach, dass die $TiO_2$-Oberflächen für den Aufbau der biologisch erkennenden Elemente der Ausführungsform A1, A2, B1 und B2 mit organischen Trägerschichten versehen werden müssen, welche das gerichtete bzw. ungerichtete Immobilisieren von Rezeptormolekülen in einer zwei- oder dreidimensionalen Anordnung erlauben.

Neben den erwähnten Anforderungen an die Dimensionen und Durchlässigkeit einer Trägerschicht, welche für die zwei- oder dreidimensionale Anordnung von Rezeptormolekülen zu stellen sind, müssen diese organischen Trägerschichten für die Immobilisierung der Rezeptormoleküle auch chemischen bzw. physicochemischen Anforderungen genügen und verfügen über

a) reaktive Gruppen, mittels derer Rezeptormoleküle kovalent an/in der zwei-/dreidimensionalen Trägerschicht verankert werden können und

b) funktionelle Gruppen, oder Moleküle und/oder Molekülverbände, welche ein effizientes Aufkonzentrieren von Rezeptormolekülen an/in dieser zwei/dreidimensionalen Matrix vor dem kovalenten Verankern ermöglichen, sodass das Immobilisieren von Rezeptormolekülen aus verdünnten Lösungen erfolgen kann.

ad a): Aus der Literatur sind eine Vielzahl reaktiver Gruppen bekannt, die für ein derartiges kovalentes Immobilisieren herangezogen werden können. Man unterscheidet zwischen Gruppen, welche als solche chemisch aktiv sind und mit funktionellen Gruppen am Rezeptormolekül eine Bindung eingehen können wie z.B. Amino-, Hydrazin- oder Hydrazidgruppen auf der Trägerschicht, welche mit Aldehydgruppen am Rezeptormolekül reagieren können und vice versa, aktivierte Disulfidbindungen auf der Trägerschicht, welche mit freien Thiolgruppen am Rezeptormolekül reagieren, Karbonsäurehalogenide oder aktivierte Karbonsäureester auf der Trägerschicht, welche mit Amingruppen auf der Oberfläche des Rezeptormoleküls reagieren etc. und Gruppen, welche nach einer in situ Aktivierung (chemische oder photochemische Aktivierung) mit funktionellen Gruppen am Rezeptormolekül reagieren wie z.B. Aziridine oder Phenylazide, welche durch eine photochemische Aktivierung in reaktive Carbene oder Nitrene überführt werden.

ad b) eine derartige aufkonzentrierende Eigenschaft kann einer Trägerschicht auf verschiedene Arten vermittelt werden, so z.B durch ionische Gruppen, durch welche Rezeptormoleküle entgegengesetzter Gesamtladung aufgrund einer Coulombwechselwirkung mit der ionischen Gruppe der Trägerschicht in annähernd ungerichteter Art und Weise aufkonzentriert werden,

oder durch Molekülverbände, welche einer Trägerschicht einen hydrophoben Charakter verleihen, sodass Rezeptormoleküle mit hydrophoben Domänen über diese Domänen an diesen Oberflächen in gerichteter Art und Weise aufkonzentriert werden,

oder durch Metallkomplexe mit nicht abgesättigter Koordinationssphäre, welche durch bestimmte funktionelle Gruppen oder Domänen eines Rezeptormoleküls abgesättigt werden und dadurch eine gerichtete Aufkonzentrierung an der Trägerschicht erfolgt,

oder durch Moleküle mit der Fähigkeit eines molekularen Erkennens (z.B. Protein A, Protein G, Streptavidin, Antikörper gegen bestimmte Epitope eines Erkennungsmoleküls etc.), welche eine hohe Affinität zu bestimmten Domänen eines Erkennungsmoleküls haben und welche ein Rezeptormolekül als Folge dieser Affinität an/in der Trägerschicht in gerichteter Art und Weise aufkonzentrieren.

Ueberraschenderweise zeigte sich, dass bei der erfindungsgemässen Beschichtung von planaren $TiO_2$-Wellenleitern organische Schichten entstehen, welche einen analogen Aufbau und einen vergleichbaren Ordnungsgrad aufweisen wie organische Monolagen, welche mit den Verbindungen der Formel II auf Materialien wie Silizium, Siliziumoxid und Aluminiumoxid (Advanced Materials 2 (1990) 573; Langmuir 8 (1992) 947) aufgebracht werden oder wie die organischen Monolagen, welche mit funktionalisierten Thioalkanen auf Gold-Oberflächen (Langmuir 6 (1990) 87) aufgebracht werden können. Die Klasse derartiger organischer Schichten wird in der Fachliteratur mit dem Begriff "self assembled monolayer" bezeichnet. Unter den geschilderten Bedingungen entstehen organische Monolagen auf den $TiO_2$-Oberflächen, bei denen die Verbindungen der Formel II über das endständige Si-Atom mit dem $TiO_2$ kovalent verknüpft sind und die Spacergruppe Y mit der reaktiven Gruppe X von der Oberfläche absteht. Die Verbindungen der Formel II binden an die $TiO_2$-Schichten über die reaktiven Gruppe $(R^1R^2R^3)$-Si-, indem mindestens eine der Gruppen $(R^1,R^2,R^3)$ mit freien Hy-

droxylgruppen auf der Oberflächen reagiert. Um eine dichte Packung und resistente Schichten zu erhalten ist es deshalb wichtig, die $TiO_2$-Oberfläche in geeigneter Art und Weise vorzubehandeln, sodass an der Oberfläche eine hohe Dichte von Hydroxylfunktionen entsteht. Die auf den $TiO_2$-Oberflächen mit den Verbindungen der Formel II erhaltenen Schichten erweisen sich in organischen Lösungsmitteln und in wässerigen Medien mit einem 9>pH>1 als stabil. In basischen wässerigen Medien pH>10 nimmt ihre Stabilität ab, und zwar mit abnehmender Anzahl reaktiver Gruppen R am endständigen Si-Atom der Verbindungen der Formel II.

Diese Monolagen sind in Bezug auf ihre Haftung an der $TiO_2$-Oberflächeauch beständig gegen Reduktionsmittel wie $BH_3$ oder $LiAlH_4$ beziehungsweise Oxidationsmittel wie wässerige Permanganatlösung oder Perchloratlösung.

Diese kovalent mit der Oberfläche des $TiO_2$-verknüpfte Monolage organischer Verbindungen kann direkt als zweidimensionale Trägerschicht eingesetzt werden, wenn es sich bei den reaktiven Gruppen um solche handelt, welche mit funktionellen Gruppen an Rezeptormolekülen reagieren (z.B. Säurehalogenid, Epoxid, Aldehyd, Hydrazid). Andernfalls müssen diese Gruppen in geeigneter Weise modifiziert (z.B. Olefin in Karbonsäure, Halogenid, Epoxid, oder Halogenid in Azid und weiter in Amin, oder Thiocyanat in Thiol) und/oder aktiviert (z.B. Karbonsäure in aktivierten Ester) werden. Derartige Verfahren für die Modifikation oder Aktivierung von funktionellen Gruppen an Oberflächen sind aus der Literatur bekannt (z.B. IEEE Transactions on Biomedical Engineering 35 (1988), 466; Analytica Chimica Acta 229 (1990) 169; Analytica Chimica Acta 228 (1990) 107; Biosensors and Bioelectronics 7 (1991) 207, Langmuir 6 (1990), 1621). Eine weitere Möglichkeit der Modifizierung besteht in der Addition von heterobifunktionellen Photoreagenzien (z.B. Verbindungen mit Phenylazido- oder Aziridino-Gruppen als photoreaktive Gruppen und einer aktivierten Karbonsäure als chemisch reaktive Gruppe) über die chemisch reaktive Gruppe an die funktionellen Gruppen X, wodurch eine Oberfläche entsteht, an welcher im Zuge einer Belichtung Rezeptormoleküle an der Oberfläche immobilisiert werden können (Journal of Photochemistry and Photobiology, B:Biology 7 (1990) 277).

Eine Variante der oben beschriebenen monofunktionellen Trägerschicht, welche zu erkennenden Elementen der Ausführungsform A führt, besteht darin, dass unter Verwendung der Verbindungen der Formel II eine Oberfläche mit unterschiedlichen funktionellen Gruppen erzeugt wird, sodass eine multifunktionelle organische Monolage entsteht. Eine derartige Mischschicht kann erzeugt werden durch Verwendung einer Mischung von Verbindungen der Formel II für die Beschichtung der $TiO_2$-Oberfläche oder aber durch eine nachfolgende chemische Modifikation bei der chemisch reaktive Gruppen X an der Oberfläche nur partiell in eine Gruppe X' umgewandelt werden. Eine derartige Mischschicht kann dann chemisch reaktive (wie z.B. aktivierte Karbonsäure) oder aktivierbare (wie z.B. Aziridin oder Phenylazid) Gruppen tragen und gleichzeitig auch funktionelle Gruppen, Moleküle und/oder Molekülverbände, welche das oben erwähnte Aufkonzentrieren der Rezeptormoleküle für das Immobilisieren erlauben.

Zum Beispiel kann in einem ersten Beschichtungsschritt eine organische Monolage erzeugt werden, welche als funktionelle Gruppen X Hydroxylgruppen trägt. Dies erfolgt in einfacher Weise durch die Behandlung der $TiO_2$ Oberfläche mit einer Verbindung der Formel II, welche als funktionelle Gruppe X eine Doppelbindung trägt. Durch Behandlung dieser Oberfläche mit Persäuren (z.B. Chlorperbenzoesäure und nachfolgende Behandlung mit sauren wässerigen Lösungen von pH = 3) wird diese Doppelbindung in eine Diol-Gruppe umgewandelt. Beim Behandeln dieser Oberfläche mit Verbindungen der Formel II in denen die Spacergruppe eine Perfluoralkankette darstellt und die funktionelle Gruppe ein Fluoratom ist entsteht eine Oberfläche, welche Rezeptormoleküle bevorzugt über hydrophobe Domänen bindet. Das dadurch entstehende erkennende Element besitzt die charakteristischen Eigenschaften eines Elementes der Ausführungsform A2 (gerichtete Immobilisierung von Rezeptormolekülen in einer zweidimensionalen Anordnung). Es wurde z.B. überraschenderweise gefunden, dass an derartigen Oberflächen aufkonzentrierte und verankerte Membranproteine bevorzugt über den Transmembranteil auf dieser Oberfläche binden und so annähernd 100% ihrer natürlichen Aktivität bewahren.

Eine alternative Modifikation derartiger organischer Monolagen besteht in der Addition von Biomolekülen, welche bestimmte, nicht Analyt-bindende Domänen der zu immobilisierenden Rezeptormoleküle erkennen und binden. Für das Aufkonzentrieren von Antikörpern kann z.B. auf eine organische Monolage über aktivierte Karboxylsäuregruppen eine Monolage Protein A immobilisiert werden. An Protein A werden unter geeigneten Pufferbedingungen die Antikörper über ihren Fc-Teil adsorbiert. Durch unspezifische Coadsorption von Molekülen, welche photoaktivierbare Gruppen tragen (z.B. mit Phenylazidoverbindungen modifiziertes BSA) können diese adsorbierten Antikörper anschliessend auf der Oberfläche in einer lichtinduzierten Reaktion verankert werden und es entsteht erneut ein erkennendes Element der Ausführungsform A2.

In einer bevorzugten Modifikation derartiger bifunktioneller Trägerschichten, welche zu erkennenden Elementen der Ausführungsform A1 führt, werden die reaktiven Gruppen der organischen Monolage dazu verwendet um niedermolekulare (MG>1500) hydrophile Moleküle an dieser Oberfläche zu verankern. Bevorzugte Vertreter dieser kurzkettigen, hydrophilen Verbindungen sind niedermolekulare Polymere wie Oligovinylalkohole, Oligoacrylsäure und Oligoacrylsäurederivate, Oligoethylenglykole oder niedermolekulare natürliche Verbindungen wie Monosaccharide, Oligosaccharide mit 2-7 Zuckereinheiten, oder Karboxyglycoside oder Aminoglycoside (wie z.B. Fradiomycin, Kanamycin, Streptomycin, Xylostasin, Butirosin, Chitosan etc.). Durch eine Addition derartiger niedermolekularer Verbindungen entstehen Trägerschichten, welche ihren zweidimensionalen Charakter noch bewahren aber gleichzeitig ein hohes Mass an Biokompatibilität aufweisen. Es hat sich überraschenderweise gezeigt, dass sich derartige niedermolekulare, hydrophile Verbindungen in ausgezeichneter Art und Weise dazu eignen zweidimensionale Trägerschichten aufzubauen an denen sich Rezeptormoleküle aus verdünnten Lösungen auikonzentrieren und verankern lassen, wenn diese Verbindungen mit ionischen Gruppen (z.B Karboxylat) und mit reaktiven Gruppen X' (z.B chemisch reaktive Gruppen wie Aldehyde, Epoxide, aktivierte Ester etc. oder photochemisch reaktive Gruppen wie Aziridin oder Phenylazid) ausgestattet werden. Auf diesem Weg entsteht dann z.B. ein erknndes Element der Ausführungsform A1.

Besonders einfach fällt eine geeignete Modifizierung derartiger hydrophiler Oberflächen im Falle der Verwendung der oben erwähnten Aminoglycoside. Diese Aminoglycoside, von denen die meisten eine antibiotische Wirkung haben, sind im allgemeinen aufgebaut aus 1-5 Zuckereinheiten, welche mit ein oder mehreren Aminogruppen derivatisiert sind. Eine dieser Aminogruppen kann dazu verwendet werden um das Aminoglycosid auf der oben beschriebenen organischen Monolage zu immobilisieren, wenn diese Monolage geeignete reaktive Gruppen (z.B. Karbonsäurehalogenid, aktivierte Karbonsäure, Aldehyd) trägt. Die restlichen Aminogruppen können so modifiziert werden, dass die Trägerschicht anschliessend die erwähnte Bifunktionalität (Auikonzentrieren, Binden) aufweist. In einem einfachen Verfahren kann z.B. an die Aminogruppen Bernsteinsäureanhydrid addiert werden. Ein Teil der dabei entstehenden freien Karbonsäurefunktionen kann durch Überführung in das N-Hydroxysuccinimidderivat für das chemische Binden modifiziert werden (alternativ können auch photochemisch aktive Gruppen addiert werden), während der nicht modifizierte Anteil freier Karbonsäurefunktionen in Form eines Karboxylates verwendet werden kann, um Rezeptormoleküle mit positiver Gesamtladung an der Trägerschicht aufzukonzentrieren. Es hat sich überraschenderweise gezeigt, dass die auf einer derartigen Trägerschicht in nicht gerichteter Art und Weise immobilisierten Rezeptormoleküle (erkennendes Element der Ausführungsform A1) im allgemeinen eine sehr viel höhere Bindungsaktivität für das Analytmolekül zeigen als Rezeptormoleküle, welche direkt auf eine mit den Verbindungen der Formel II dargestellten Trägerschicht immobilisiert werden.

Niedermolekulare, hydrophile Verbindungen wie Mono- und Oligosaccharide, welche in ihrer ursprünglichen Form weder reaktive Gruppen für das gerichtete Immobilisieren noch funktionelle Gruppen, Moleküle oder Molekülverbände für das Aufkonzentrieren tragen, können in geeigneter Weise modifiziert werden, wobei eine derartige Modifikation im allgemeinen effizienter erfolgt, wenn sie nicht an der schon auf der Oberfläche verankerten Verbindung erfolgt.

Ein typisches Vorgehen sei am Beispiel von Dextran 1500 (7 Glucose-Untereinheiten) demonstriert. Dieses Dextran kann in Lösung (z.B.DMSO) durch Überführung der Hydroxylgruppen in Hydroxylatgruppen effizient für eine Methylkarboxylierung aktiviert werden. Da diese Aktivierung unter Verwendung von NaH in sehr basischem Medium verläuft, ist sie aufgrund der oben erwähnten Instabilität der organischen Monolagen auf $TiO_2$ nicht direkt an der Festphase durchführbar. Das extern methylkarboxylierte Dextran 1500 kann aber auf einer organischen Monolage, welche z.B. Epoxidgruppen trägt sehr einfach verankert werden, wodurch man zweidimensionale Trägerschichten mit einer hohen Konzentration an Karboxylgruppen erhält, welche in analoger Weise für den Aufbau von erkennenden Elementen der Ausführungsform A1 verwendet werden können wie die mit Arminoglycoside und Bernsteinsäure modifizierten organischen Monolagen.

Solche unter Verwendung dieser niedermolekularen, hydrophilen Verbindungen aufgebauten Trägerschichten können auch weiter zu Trägerschichten modifiziert werden um ein Aufkonzentrieren in gerichteter Art und Weise zu erreichen (erkennende Elemente der Ausführungsform A2).

Zum Beispiel können die über Aminoglycoside oder Oligosaccharide eingeführten Karboxylgruppen dazu verwendet werden um Biomoleküle (z.B. Protein A, Protein G, Streptavidin etc) auf der Oberfläche zu verankern, welche eine hohe Affinität haben zu einer Domäne des nachfolgend zu immobilisierenden Rezeptormoleküls.

Neben der Verwendung als zweidimensionale Trägerschicht für Rezeptormoleküle sind diese bis dahin besprochenen organischen Schichten auch geeignet als Basis für den Aufbau von dreidimensionalen Trägerschichten, welche zu den erkennenden Elementen der Ausführungsform B führen.

Die Überführung der zweidimensionalen Trägerschicht in eine dreidimensionale Trägerschicht erfolgt durch die Addition langkettiger synthetischer oder natürlicher, hydrophiler Polymere, welche in der Lage sind, auf der Oberfläche des Transducers eine poröse, dreidimensionale Matrix in der Art eines Hydrogels auszubilden. Typische Vertreter geeigneter natürlicher Polymere sind z.B. Polysaccharide wie Dextran, Agarose, Algininsäure, Stärke, Zellulose oder Derivate derartiger Polysaccharide wie methylkarboxylierte Derivate oder aber synthetische, hydrophile Polymere wie Polyvinylalkohol, Polyacrylsäure, Polyethylenglykol.

Wichtig ist, dass diese langkettigen Polymere wie die niedermolekularen, hydrophilen Verbindungen ebenfalls mit reaktiven Gruppen X ausgestattet sind, welche ein Verankern von Rezeptormolekülen an dieser dreidimensionalen Trägerschicht erlauben. In einer bevorzugten Ausführungsform trägt diese Trägerschicht zudem ionische Gruppen oder ausrichtende Moleküle und /oder Molekülverbände, welche das Aufkonzentrieren von Rezeptormolekülen in ungerichteter (Ausführungsform B1) oder gerichteter Art und Weise (Ausführungsform B2) ermöglichen.

Zum Beispiel kann Dextran 500000 methylkarboxyliert und anschliessend auf einer organischen, mit Epoxidgruppen modifizierten Monolage verankert werden. Dadurch entsteht eine ca. 100 nm dicke, poröse Trägerschicht mit Karbonsäuregruppen von denen ein Teil als Karboxylat die Aufkonzentration von Biomolekülen mit positiver Gesamtladung ermöglicht und der andere Teil, in aktivierter Form, für die nachfolgende kovalente Verankerung eingesetzt werden kann.

Das erfindungsgemässe biologisch erkennende Element kann gewünschtenfalls via eine dünne Zwischenschicht (d < 20 nm) von $SiO_2$ oder $Al_2O_3$ auf den optischen $TiO_2$-Wellenleiter aufgebracht werden.

Die folgenden Beispiele erläutern die Erfindung näher:

1. Aufbringen von dicht gepackten, organischen Monolagen auf $TiO_2$-Wellenleiter

1.1. Ausbildung einer organischen Monolage auf $TiO_2$-Oberflächen durch Behandlung mit Cl-$(CH_3)_2$Si-$(CH_2)_{11}$-COCl in einem CVD Verfahren:

Für das Aufbringen der Verbindungen der Formel II aus der Gasphase wird ein Reaktionsgefäss bereitgestellt , welches bei einem Druck von 10-5 mbar betrieben werden kann und in welchem die zu beschichtende Probe auf Temperaturen zwischen 30-100°C gebracht werden kann. Dieses Reaktionsgefässs wird mit einem evakuierbaren, beheizbaren Vorratsgefäss verbunden in dem die für die Beschichtung verwendete Verbindung vorgelegt werden kann (wahlweise kann die Apparatur auch mit mehreren derartigen Vorratsgefässen ausgestattet werden).

Für die Beschichtung wird das Substrat in das Reaktionsgefäss eingebracht. Nach Einbringen des Silans $(Cl(CH_3)_2$Si-$(CH_2)_{11}$-COCl) in das Vorratsgefäss werden Vorratsgefäss und Reaktionskammer auf einen Arbeitsdruck von 10-5 mbar gebracht. Die zu beschichtende Probe wird auf 100°C erwärmt. Nach dem Aufwärmen des im Vorratsgefäss vorgelegten Reagenzes auf 50°C wird die Oberfläche während 1h mit Reagenz aus der Gasphase behandelt. Anschliessend wird der Reagenzzufluss gestoppt und die Probe im Vakuum bei 150°C während 15 min nachbehandelt.

(Der Nachweis einer organischen Monolage auf der Oberfläche erfolgt über XPS- und Kontaktwinkel-Messungen).

1.2. Ausbildung einer organischen Monolage auf $TiO_2$-Oberflächen durch Behandlung mit Cl-$(CH_3)_2$Si-$(CH_2)_6$-CH=$CH_2$ in einem CVD-Verfahren:

Für die Umsetzung der Oberfläche wird das unter 1.1 geschilderte Verfahren eingesetzt.

1.3. Ausbildung einer organischen Monolage auf $TiO_2$-Oberflächen durch Behandlung mit $(CH_3O)_3$Si-$(CH_2)_3$-$NH_2$ in einem CVD Verfahren:

Die Beschichtung verläuft unter Verwendung der entsprechenden Verbindung nach dem unter 1.1 geschilderten Verfahren.

1.4. Ausbildung einer organischen Monolage auf $TiO_2$ durch Behandlung mit einer Lösung von $Cl(CH_3)_2$Si-$(CH_2)_{11}$-COCl:

In einem Reaktionsgefäss wird unter Inertgasatmosphäre eine 0.5% (v/v) Lösung von Cl-$(CH_3)_2$Si-$(CH_2)_{11}$COCl in $CCl_4$ vorgelegt. Die zu beschichtende Oberfläche wird während 25 min unter Inertgas mit dieser Lösung in Kontakt gebracht. Nach dieser Behandlung wird die Oberfläche mit $CCl_4$, Ethanol und Wasser gereinigt.

1.5. Ausbildung einer organischen Monolage auf $TiO_2$ durch Behandlung mit einer Lösung von $Cl_3$Si-$(CH_2)_6$-CH=$CH_2$:

In einem Reaktionsgefäss wird unter Inertgasatmosphäre eine 0.5% Lösung (v/v) von $Cl_3$Si-$(CH_2)_6$-CH=$CH_2$ in Hexadekan bereitgestellt. Die zu beschichtende Oberfläche wird unter Inertgas mit dieser Lösung während 5min in Kontakt gebracht. Nach dieser Behandlung wird die Oberfläche mit Hexadekan, Hexan und Ethanol gewaschen.

1.6 Ausbildung einer organischen Monolage auf $TiO_2$ durch Behandlung mit einer Lösung von $Cl(CH_3)_2$Si-$(CH_2)_7$-$(CH_2$-O-$CH_2)_2$-$CH_2$-O-$CH_3$:

Die Beschichtung erfolgt nach dem unter 1.4 beschriebenen Verfahren.

1.7. Ausbildung einer organischen Monolage auf TiO$_2$ durch Behandlung der Oberfläche mit einer Lösung von Cl$_3$Si-(CH$_2$)$_8$Br:

Die Beschichtung erfolgt nach dem unter 1.4 beschriebenen Verfahren.

2.Modifikation von funktionellen Gruppen an organischen Monolagen auf TiO$_2$ dargestellt nach den unter 1 beschriebenen Verfahren.

2.1. Umwandlung von Olefinen in Epoxide:

Eine nach Vorschrift 1.2 oder 1.5 behandelte TiO$_2$-Oberfläche wird bei 4°C während 24 h kontaktiert mit einer Lösung von 3-Chlorperbenzoesäure (0.06 M) in Diethylether (abs). Anschliessend wird die Oberfläche mit Diethylether, Ethanol und Wasser (4°C) gewaschen.

2.2. Umwandlung von Epoxiden in Diole:

Die unter 2.1. dargestellte Oberfläche mit Epoxidfunktionen wird während 1h bei 80°C mit einer wässerigen Lösung von pH = 2.5 behandelt und anschliessend mit H$_2$O gereinigt.

2.3. Umwandlung von Olefinen in Karbonsäuren.

Die nach Vorschrift 1.2 oder 1.5 behandelten TiO$_2$-Oberflächen werden mit einer wässerigen Lösung von Kaliumpermanganat (0.1M) und NaJO$_4$ (0.1M) während 20 min in Kontakt gebracht. Anschliessend wird die Oberfläche mit 0.1M wässeriger NaHSO$_3$, mit Ethanol und Wasser gewaschen.

2.4. Umwandlung von Halogeniden in Azide.

Eine nach der Vorschrift 1.7 behandelte TiO$_2$-Oberfläche wird mit einer Lösung von NaN$_3$ (6 mg/ml) in DMF (abs.) während 15 h in Kontakt gebracht und anschliessend mit DMF und Wasser gewaschen.

2.5. Umwandlung von Aziden in Amine.

Die nach der Vorschrift 2.4 dargestellte TiO$_2$-Oberfläche mit N$_3$-Gruppen wird während 4h mit einer Lösung von SnCl$_2$ in absolutem Methanol in Kontakt gebracht. Die Oberfläche wird anschliessend mit Methanol und Wasser gewaschen.

2.6. Aktivierung von Karbonsäuren mit Chlorameisensäureethylester und N-Hydroxysuccinimid:

Die nach Vorschrift 2.3 dargestellte Oberfläche mit COOH-Gruppen wird während 1h mit einer 2.5% Lösung (v/v) von Chlorameisensäureethylester in CH$_2$Cl$_2$/Pyridin (100/2.5) in Kontakt gebracht. Anschliessend wird die Oberfläche mit einer Lösung von N-Hydroxysuccinimid (0.5M) in Pyridin kontaktiert. Es entstehen dadurch N-Hydroxysuccinimid-aktivierte Karbonsäurefunktionen an welche Moleküle mit Aminogruppen direkt addiert werden können.

3. Modifizierung von organischen Monolagen dargestellt nach den unter 2 beschriebenen Verfahren mit niedermolekularen, hydrophilen Verbindungen.

3.1. Addition von Fradiomycin an eine mit einer organischen Monolage ausgestatten TiO$_2$-Oberfläche.

Die nach Vorschrift 2.6. dargestellte Oberfläche mit aktivierten Karbonsäurefunktionen wird während 1h mit einer Lösung von Fradiomycin (20 mM in PBS; pH = 7.2) in Kontakt gebracht. Anschliessend wird mit H$_2$O gewaschen.

3.2. In situ Modifikation von immobilisiertem Fradiomycin

a) Einführen von Karbonsäurefunktionen: Die Aminogruppen des nach Vorschrift 2.1 auf der Oberfläche immobilisierten Fradiomycins werden durch den Kontakt mit einer Lösung (1% w/w) von Bernsteinsäureanhydrid in Pyridin quantitativ umgesetzt. Dadurch wird eine hydrophile Trägerschicht erzeugt, die eine hohe Dichte verfügbarer Säurefunktionen aufweist.

b) Einführen aktiver Disulfidbindungen: An die Aminogruppen des nach Vorschrift 3.1 auf der Oberfläche immobilisierten Fradiomycins kann durch den Kontakt mit einer ethanolischen Lösung (2 mM) von N-Succinimidyl 3-(2-pyridinyldithio)propionat 3-(2-pyridinyl)-dithiopropionat angekoppelt werden. Die Dithiopyridinylgruppe kann verwendet werden, um Moleküle (z.B. Fab'-Fragmente von IgG-Molekülen) gezielt über freie Thiolfunktionen an der Trägerschicht zu immobilisieren. Die bei diesem Verfahren nicht umgesetzten Aminogruppen können durch das unter a) beschriebene Verfahren dazu verwendet werden, um Karbonsäurefunktionen auf der Oberfläche zu immobilisieren.

c) Einführen photoaktiverbarer Phenylazidogruppen: An die Aminogruppen des nach Vorschrift 3.1. auf der Oberfläche immobilisierten Fradiomycins kann durch den Kontakt mit einer wässerigen (10% DMSO) Lösung ( 2 mM) von N-Succinimidyl 6-(4'-azido-2'-nitrophenylamino)hexanoat immobilisiert werden. Nicht umgesetzte Aminfunktionen können nach dem unter a) beschriebenen Verfahren dazu verwendet werden um die Trägerschicht gleichzeitig auch mit Karbonsäuregruppen zu modifizieren.

4. Aufbau einer porösen Trägerschicht auf den organischen Monolagen für die Herstellung dreidimensionaler erkennender Elemente:

4.1.Methylkarboxylierung von Dextran 500000

Zu 7.5g NaH werden 75 ml trockenes DMSO zugegeben. Die Konzentration dabei entstehender DMSO-Anionen wird durch Titration bestimmt. 0.2 Equivalente (bezogen auf Glucoseuntereinheiten) Dextran 500000 wird in 150 ml trockenem DMSO gelöst und die Lösung mit DMSO-Anionen zugegeben. Es wird während 4 h bei Raumtemperatur gerührt und anschliessend ein zweifacher Überschuss (bezogen auf Glucoseuntereinheiten) an Bromessigsäure zugegeben. Die Lösung wird 12 h gerührt. Anschliessend wird das Dextran mit Aceton ausgefällt, abfiltriert, in 20 ml Wasser gelöst und während 24 h gegen Wasser dialysiert. Nach dem Lyophilisieren wird der Anteil an methylkarboxylierten Glucoseuntereinheiten durch Titration bestimmt (ca 1 Karboxylgruppe/5 Glucoseuntereinheiten).

4.2. Immobilisierung von methylkarboxyliertem Dextran 500000 auf organischen Monolagen.

Die Immobilisierung von methylkarboxyliertem Dextran geht aus von organischen Monolagen welche nach Vorschrift 1.7 auf $TiO_2$-Oberflächenerzeugt und nach den Vorschriften 2.4 und 2.5 modifiziert wurden. Die Aminogruppen dieser organischen Monolagen werden mit Epichlorhydrinlösung (1ml Epichlorhydrin in 10ml NaOH(0.4M)/10ml Diglyme) umgesetzt. Nach dem Waschen mit Ethanol und Wasser wird während 24 h mit einer Lösung von methylkarboxyliertem Dextran behandelt (0.3g Dextran in wässeriger NaOH-Lösung (0.01 M NaOH). Anschliessend wird die Oberfläche mit Wasser bei 50 °C gut gewaschen.

5. Darstellung von erkennenden Elementen der Ausführungsform A1, A2, B1 und B2 auf der Basis der in den Abschnitten 1-4 besprochenen Trägerschichten.

5.1 Darstellung von erkennenden Elementen der Ausführungsform A1 mit immobilisiertem IFNα - (Interferon α) als Rezeptormolekül.

Für die Immobilisierung von IFNα wird eine Trägerschicht hergestellt welche mit Bernsteinsäuranhydrid modifiziert wurde (Beschichtung von $TiO_2$ nach 1.1, Addition von Fradiomycin nach 3.1., Modifikation des Fradiomycins nach 3.2.a.). Diese Oberfläche wird mit einer wässerigen Lösung von N-(3-dimethylaminopropyl)-N'-ethylcarbodiimid (20mg/ml) und N-hydroxysuccinimid (3 mg/ml) während 5 min behandelt. Nach dem Waschen mit Acetatpuffer (0.01 M; pH = 5.5) wird mit einer Lösung von Interferon (0.9 μg/ml) während 20 min inkubiert. Die über dieses Prozedere erreichte Oberflächenkonzentration an IFNα beträgt nach dem Waschen mit Acetatpuffer und 0.01 M HCl 0.36 ng/mm$^2$.

5.2.Darstellung eines erkennenden Elementes auf $TiO_2$ nach Ausführungsform A2 mit GpIIb-IIIa (Glykoprotein IIb-IIIa) als Rezeptormolekül:

Für die Immobilisierung des GpIIb-IIIa geht man aus von einer $TiO_2$ Schicht welche mit einer Diol-haltigen Oberfläche modifiziert ist (Darstellung nach Vorschrift 1.5, 2.1 und 2.2). Diese Oberfläche wird behandelt mit einer 0.5% Lösung von 1H,1H,2H,2H-Perfluoroctyldimethyl-

chlorsilan in $CCl_4$ behandelt. Die dabei entstehende stark hydrophobe Oberfläche wird mit einer wässerigen Lösung von GpIIb-IIIa (0.5 μg/ml) (0.1M Tris; pH = 7.2) während 20 min in Kontakt gebracht. Die über dieses Verfahren erreicht Oberflächenkonzentration an GpIIb-IIIa beträgt nach dem Waschen mit Puffer-Lösung 1.5 ng/mm$^2$.

5.3.Darstellung eines erkennenden Elementes auf $TiO_2$ nach Ausführungsform B1 mit TNFα - (Tumor Nekrose Factor α) als Rezeptormolekül:

Für die Immobilisierung von TNFα wird eine nach Vorschrift 1.5, 4.2 mit methylkarboxyliertem Dextran modifizierte $TiO_2$-Oberfläche verwendet. Diese Oberfläche wird mit einer wässerigen Lösung von N-(3-dimethylaminopropyl)-N'-ethylcarbodiimid (20mg/ml) und N-hydroxysuccinimid (3 mg/ml) während 5 min behandelt. Nach dem Waschen mit Acetatpuffer (0.01 M, pH = 5.5) wird mit einer Lösung von TNFα in Acetatpuffer 1μg/ml während 10 min inkubiert. Nach diesem Verfahren sind nach dem Waschen mit Acetatpuffer, PBS-Puffer (0.1 M; pH = 7.2) und Ethanolamin 1 M, pH = 8.5) ca. 2 ng/mm$^2$ TNFα kovalent immobilisiert.

5.4.Darstellung eines erkennenden Elementes auf $TiO_2$ nach Ausführungsform B2 mit Antikörpern als Rezeptormoleküle:

Das gerichtete Immobilisieren der Antikörper erfolgt auf einer Dextranträgerschicht, welche nach Vorschrift 1.5, 4.2 dargestellt wurde. Für das gerichtete Immobilisieren werden nach an sich bekannten Verfahren an den Karbohydratresten der Antikörper durch Oxidation freie Aldehydfunktionen erzeugt. Die Dextranschicht auf dem $TiO_2$-Wellenleiter wird mit einer wässerigen Lösung von N-(3-dimethylaminopropyl)-N'-ethylcarbodiimid (20mg/ml) und N-hydroxysuccinimid (3 mg/ml) während 5 min behandelt. Nach dem Waschen mit Wasser werden die aktivierten Karbonsäurefunktionen an der Oberfläche durch den Kontakt mit einer wässerigen Lösung von Hydrazin-Monohydrochlorid (1 mM) zu Hydraziden umgesetzt. Diese Oberfläche wird während 20 min mit einer Lösung der oxidativ behandelten Antikörper (1 μg/ml in Acetatpuffer (0.01 M, pH = 5.5)) in Kontakt gebracht. Die Oberfläche wird mit PBS (0.1 M; pH = 7.2) und einer wässerigen Lösung von Ethanolamin (1 M; pH = 8.5) gewaschen. Dieses Verfahren führt zu einer gerichteten Immobilisierung von ca. 5 ng/mm$^2$ Antikörper auf der Dextran-Trägerschicht.

**Patentansprüche**

1.  Optischer Biosensor bestehend aus einem dielektrischen Wellenleiter und einer organischen Trägerschicht an welche Rezeptormoleküle gebunden sind, dadurch gekennzeichnet, dass die organische Trägerschicht eine monomolekulare Schicht bildet, wobei die Trägerschicht aus Molekülen der allgemeinen Formel I

$$\geqslant Si - Y - Z \qquad I$$

besteht und die Moleküle der Trägerschicht über das Si-Atom direkt an einen $TiO_2$-Wellenleiter oder gewünschtenfalls indirekt über eine Zwischenschicht an einen $TiO_2$-Wellenleiter gebunden sind.

2. Optischer Biosensor nach Anspruch 1, dadurch gekennzeichnet, dass die Gruppe Y eine Spacergruppe darstellt und es sich bei den Gruppen Z um Hydroxyl-, Karboxyl, Amin- oder um Methyl-, Alkyl- oder Fluoralkyl-Gruppen handelt.

3. Optischer Biosensor nach einem der Ansprüche 1-2, dadurch gekennzeichnet, dass es sich bei den Gruppen Z um Derivate von hydrophilen, kurzkettigen Molekülen wie Oligovinylalkohole, Oligoacrylsäuren, Oligoacrylsäurederivate, Oligoethylenglykole oder um mono- oder oligo-Saccharide mit 1-7 Zuckereinheiten oder um Karboxyglycoside oder Arninoglycoside mit 1-5 Zuckereinheiten handelt.

4. Optischer Biosensor nach einem der Ansprüche 1-3, dadurch gekennzeichnet, dass es sich bei den Aminoglycosiden um Derivate von Verbindungen wie Fradiomycin, Kanamycin, Streptomycin, Xylostasin, Butirosin oder Chitosan handelt.

5. Optischer Biosensor nach einem der Ansprüche 1-4, dadurch gekennzeichnet, dass es sich bei den Gruppen Z um hydrogelbildende Gruppen handelt.

6. Optischer Biosensor nach einem der Ansprüche 1-5, dadurch gekennzeichnet, dass es sich bei den hydrogelbildenden Gruppen um Derivate von Polysacchariden wie Dextran, Agarose, Algininsäure, Stärke, Zellulose und Derivate derartiger Polysaccharide oder um hydrophile Polymere wie Polyvinylalkohole, Polyacrylsäuren, Polyethylenglykole und ihre Derivate handelt.

7. Optischer Biosensor nach einem der Ansprüche 1-6, dadurch gekennzeichnet, dass es sich bei der Zwischenschicht um eine dünne Schicht (d< 20 nm) von $SiO_2$ oder $Al_2O_3$ handelt.

8. Verfahren zur Herstellung eines optischen Biosensors nach einem der Ansprüche 1-7, dadurch gekennzeichnet, dass für die Herstellung der geordneten monomolekularen Schicht, Verbindungen der allgemeinen Formel II

$$(R^1R^2R^3)Si-Y-X \qquad II$$

verwendet werden, wobei X Wasserstoff, Fluor oder eine chemisch reaktive Gruppe ist, $R^1R^2R^3$ Alkyl, Alkoxy oder Halogen ist, Y eine Spacergruppe ist und dass diese Verbindungen entweder aus der Gasphase oder aus Lösung auf den $TiO_2$-Wellenleiter aufgebracht werden und gegebenenfalls die Gruppe X nachträglich durch Oxidation, Reduktion, Substitution oder Addition so verändert wird, dass die Gruppe Z entsteht und an die Gruppe Z das Rezeptormolekül angekoppelt werden kann.

9. Verfahren zur Herstellung eines optischen Biosensors nach Anspruch 8, dadurch gekennzeichnet, dass die Gruppe Z durch Addition, Substitution, Oxidation oder Reduktion so verändert wird, dass sie Gruppen, Moleküle oder Molekülverbände trägt, welche das Aufkonzentrieren von Rezeptormolekülen in gerichteter oder ungerichteter Art und Weise erlauben.

10. Verfahren zur Herstellung eines optischen Biosensors nach einem der Ansprüche 8-9, dadurch gekennzeichnet, dass die Gruppe Z durch Addition, Substitution, Oxidation oder Reduktion so verändert wird, dass sie anschliessend photoreaktive Gruppen trägt.

11. Verwendung eines optischen Biosensors, nach einem der Ansprüche 1-10 zur Bestimmung von Konzentrationen eines Analytmoleküls in Lösung oder zur Quantifizierung einer Wechselwirkung zwischen Analyt und Rezeptormolekül auf der Basis von thermodynamischen und kinetischen Daten.

Europäisches Patentamt

**EUROPÄISCHER RECHERCHENBERICHT**

Nummer der Anmeldung

EP 93 11 7547

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| P,X | WO-A-92 21976 (FISONS PLC.)<br>* das ganze Dokument *<br>--- | 1-3,5-11 | G01N21/55<br>G01N33/551<br>G01N33/547 |
| X | WO-A-92 03720 (FISONS PLC.)<br>* das ganze Dokument *<br>--- | 1-3,7-11 | |
| A | EP-A-0 416 730 (HEWLETT-PACKARD COMPANY.)<br>* das ganze Dokument *<br>--- | 1-11 | |
| A | EP-A-0 175 585 (CORNING GLASS WORKS.)<br>* das ganze Dokument *<br>--- | 1-11 | |
| A | EP-A-0 395 137 (ENIRICERCHE S. P. A.)<br>* das ganze Dokument *<br>--- | 1-11 | |
| A | EP-A-0 092 688 (SAGAX INSTRUMENT AB.)<br>* das ganze Dokument *<br>----- | 1-11 | |

RECHERCHIERTE SACHGEBIETE (Int.Cl.5)

G01N
C12Q

Der vorliegende Recherchenbericht wurde für alle Patentansprüche erstellt

| Recherchenort | Abschlußdatum der Recherche | Prüfer |
|---|---|---|
| DEN HAAG | 23. Februar 1994 | Griffith, G |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes
    Dokument

EPO FORM 1503 03.82 (P04C03)